**Europäisches Patentamt**

**European Patent Office**

**Office européen des brevets**

(19)

(11) Veröffentlichungsnummer: **0 134 444**

**A1**

(12)

# EUROPÄISCHE PATENTANMELDUNG

(21) Anmeldenummer: **84107300.0**

(22) Anmeldetag: **26.06.84**

(51) Int. Cl.⁴: **C 07 D 241/44**
**A 23 K 1/16**

---

(30) Priorität: **09.07.83 DE 3324908**

(43) Veröffentlichungstag der Anmeldung:
**20.03.85 Patentblatt 85/12**

(84) Benannte Vertragsstaaten:
**AT BE CH DE FR GB IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Voege, Herbert, Dr.**
**Martin-Buber-Strasse 41**
**D-5090 Leverkusen 3(DE)**

(72) Erfinder: **Sieveking, Hans Ulrich, Dr.**
**Walter-Flex-Strasse 23**
**D-5090 Leverkusen(DE)**

---

(54) **Stabilisiertes 2-N-(2-Hydroxyethyl)-carbamoyl-3-methyl-chinoxalin-1,4-N-dioxid.**

(57) Die Erfindung betrifft (2-[N-Hydroxyethyl]-carbamoyl]-3-methyl-chinoxalin-1,4-N-dioxid, das in der Korngrößenverteilung bei einem Durchmesser von 0,02 bis 1 mm ein Maximum aufweist und dadurch stabilisiert ist bei der Verarbeitung.

EP 0 134 444 A1

0134444

BAYER AKTIENGESELLSCHAFT          5090 Leverkusen, Bayerwerk

Konzernverwaltung RP
Patentabteilung                   Ad/Ke-c


Stabilisiertes 2-[N-(2-Hydroxyethyl)-carbamoyl]-3-
methyl-chinoxalin-1,4-N-dioxid

Die Erfindung betrifft durch eine definierte Korngrößenverteilung stabilisiertes 2-N-[N-(2-Hydroxyethyl)-carba-
moyl]-3-methyl-chinoxalin-1,4-N-dioxid sowie dessen Herstellung.

Üblicherweise werden Futtermittelzusätze in Form eines Prämix in den Handel gebracht. Dieses Prämix wird mit dem entsprechenden Futter gemischt. Der Wirkstoff liegt im Endfutter im ppm-Bereich vor. Dieses Endfutter wird in Pulverform, sehr häufig aber auch in Form von Pellets, Granulaten oder Blöcken an die Tiere verfüttert.

Es ist von vielen Substanzen z.B. Vitaminen bekannt, daß sie im Futter nicht unbegrenzt stabil sind und auch bei der Futtermittelherstellung insbesondere der Pellet- und Granulat-Herstellung treten Verluste durch die Wärmeentwicklung bei der Verarbeitung durch Dampfkonditio- nierung und Reibungswärme auf.

Le A 22 397

- 2 -

0134444

Bei dem Wirkstoff 2-/N̄-(2-Hydroxyethyl)-carbamoyl̲/-3-methylchinoxalin-1,4-N-dioxid (Olaquindox) traten diese Schwierigkeiten ebenfalls auf. Der eingesetzte Wirkstoff lag dabei in einer Partikelfeinheit von vorwiegend 10 mcm vor.

Überraschenderweise wurde nun gefunden, daß der Wirkstoff praktisch nicht abgebaut wird aber trotzdem noch genügend homogen verteilt vorliegt, wenn die Partikelgröße soweit erhöht wird, daß bei der Korngrößenverteilung das Maximum der Kornverteilung zwischen 0,02 - 1 mm, vorzugsweise aber zwischen 0,02 - 0,5 mm liegt.

Die Erfindung betrifft daher 2-/N̄-(2-Hydroxyethyl)-carbamoyl̲/-3-methyl-chinoxalin-1,4-N-dioxid, das dadurch charakterisiert ist, daß in seiner Korngrößenverteilung die Anzahl der Partikel mit einem Durchmesser von 0,02 bis 1 mm ein Maximum aufweist.

Liegt die Korngrößenverteilung des Wirkstoffes im Maximum über 0,5 mm, beginnen Probleme mit der Homogenität der Endmischung und der Probenentnahme.

Zur Herstellung des erfindungsgemäßen 2-/N̄-(2-Hydroxyethyl)-carbamoyl̲/-3-methyl-chinoxalin-1,4-N-dioxid können verschiedene Verfahren benutzt werden. So ist es möglich,

a)   den Wirkstoff auf den erfindungsgemäßen Korngrößenbereich zu fällen

b)   den Wirkstoff auf den genannten Korngrößenbereich zu mahlen

Le A 22 397

c)  den Wirkstoff durch Agglomeration oder Granulation
feinerer Kristalle einzustellen.

Der erfindungsgemäße Wirkstoff wird zur Herstellung von
Prämixen, Futtermittelzusätzen und Futtermitteln eingesetzt. Die Futtermittel können zu Pulvern, stranggepreßten
Formkörpern, Blöcken, Granulaten oder Pellets verarbeitet
werden. Gewünschtenfalls wird den Futtermitteln oder
den Vormischungen 2-/N̄-(2-Hydroxyethyl)-carbamoyl̲/-3-
methylchinoxalin-1,4-N-dioxid in Kombination mit weiteren
antibiotischen oder nicht-antibiotischen ergotropen Substanzen zugesetzt.

Der große Vorteil der geänderten Korngröße des Wirkstoffes im Hinblick auf seine Stabilität im Endfutter geht
aus folgenden Stabilitätsdaten hervor.

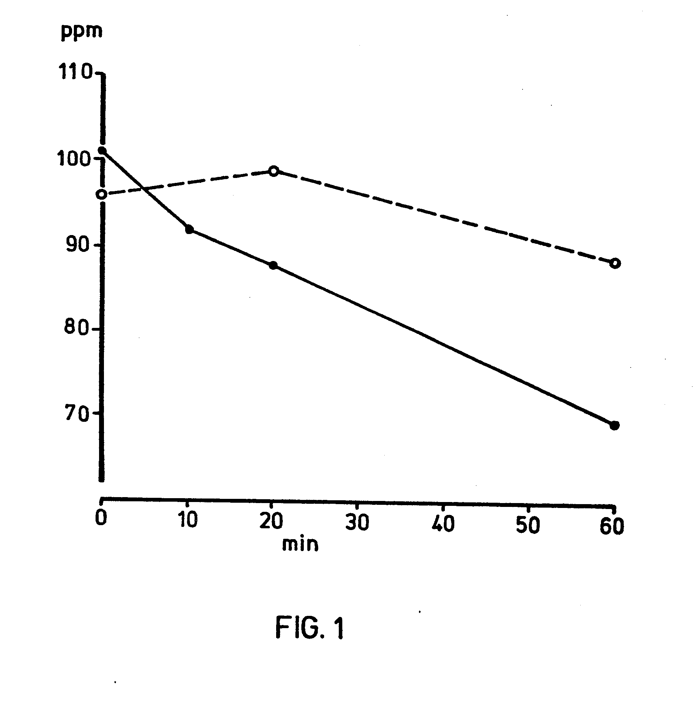

Durch 3 % Wasserzusatz und anschließende thermische Belastung bei 90°C im geschlossenen Gefäß sollte die (normalerweise vor der Pelletierung von Futtermitteln durchgeführte) Konditionierung simuliert werden. Der Wert nach
60 Min. entspricht für den feinkörnigen Wirkstoff den
Praxisbedingungen beim Pelletieren. Beim Erwärmen wurden
alle 10 Min. Muster entnommen und analysiert. Das Ergebnis ist in Abb. 1 zu sehen. (In der Abbildung ist der Wirkstoffgehalt gegen die Zeit aufgetragen. Die gestrichelte
Linie zeigt den Einfluß der Konditionierung auf den erfindungsgemäßen Wirkstoff. Die ausgezogene Linie steht für
den Wirkstoff mit einem Korngrößenverteilungsmaximum von
$\leq$ 10 mcm.). Es zeigt sich, daß der Wirkstoff gemäß Erfindung um ca. 5 % abbaut, während der feinere, körnige Wirkstoff ($\leq$ 10 mcm) ca. 30 % Aktivitätsverlust aufweist.

Le A 22 397

- 4 -

In einem Parallelversuch wurden 50 ppm Wirkstoff gemäß
Erfindung und der feinere Wirkstoff in ein Standardfutter
eingemischt (Ferkelaufzuchtfutter). Der Gehalt wurde nach
Abmischen sowie nach 5 Monaten bestimmt.

Wirkstoff gemäß Erfindung          Wirkstoff ≃10 mcm

| | Wirkstoff gemäß Erfindung | Wirkstoff ≃10 mcm |
|---|---|---|
| Anfangsgehalt | 48,7 ppm | 45,4 ppm |
| 5 Monate gelagert | 47,2 ppm | 39,2 ppm |
| | (=97% v. Anfang) | (=86% v. Anfang) |

Aus beiden Beispielen wird somit ersichtlich, welchen
Vorteil die Korngrößenbegrenzung für die Haltbarkeit des
Wirkstoffes beim Pelletieren und Lagern bringt.

Der erfindungsgemäße Wirkstoff kann in allen Bereichen
der Tierzucht als Mittel zur Förderung und Beschleunigung
des Wachstums und zur Verbesserung der Futterverwertung
bei gesunden und kranken Tieren verwendet werden.

Die Wirksamkeit des Wirkstoffs ist hierbei weitgehend
unabhängig von der Art und dem Geschlecht der Tiere.
Besonders wertvoll erweist sich der Wirkstoff bei der
Aufzucht und Haltung von Jung- und Masttieren.
Als Tiere, bei denen der Wirkstoff zur Förderung und
Beschleunigung des Wachstums und zur Verbesserung der
Futterverwertung eingesetzt werden kann, seien beispielsweise folgende Nutz- und Ziertiere genannt:

Warmblüter wie Rinder, Schweine, Pferde, Schafe, Ziegen, Katzen, Hunde, Kaninchen; Pelztiere, z.B. Nerze,

Le A 22 397

0134444

und Chinchilla; Geflügel, z.B. Hühner, Gänse, Enten, Truthähne, Tauben, Papageien und Kanarienvögel und Kaltblüter wie Fische, z.B. Karpfen und Reptilien, z.B. Schlangen.

Die Mengen des Wirkstoffs, die den Tieren zur Erreichung des gewünschten Effektes verabreicht wird, kann wegen der günstigen Eigenschaften des Wirkstoffs weitgehend variiert werden. Sie liegt im Endfutter zwischen 5 - 500 ppm, vorzugsweise zwischen 10 - 300 ppm pro Tag. Die Dauer der Verabreichung kann von wenigen Stunden oder Tagen bis zu mehreren Jahren betragen. Die zu verabreichende Menge des Wirkstoffs sowie die entsprechende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Der Wirkstoff wird den Tieren nach den üblichen Methoden über das Futter verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral erfolgen. Aus Zweckmäßigkeitsgründen ist in den meisten Fällen eine orale Verabreichung, insbesondere im Rhythmus der Nahrungsaufnahme der Tiere, vorzuziehen.

Der Wirkstoff wird in formulierter Form, also in Mischung mit nichttoxischen inerten Trägerstoffen beliebiger Art,

Le A 22 397

z.B. mit Trägerstoffen und in Formulierungen, wie sie bei nutritiven Zubereitungen üblich sind, verabreicht.

Der Wirkstoff wird gegebenenfalls in formulierter Form zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht.

Empfehlenswert ist die orale Verabreichung zusammen mit dem Futter, wobei je nach Bedarf der Wirkstoff der Gesamtmenge oder nur Teilen des Futters zugegeben wird.

Der Wirkstoff wird nach üblichen Methoden durch einfaches Mischen mit eßbaren nichttoxischen Trägerstoffen,gegebenenfalls in Form eines Prämix oder eines Futterkonzentrates, zu einem Endfutter verarbeitet.

Das Futter kann beispielsweise den Wirkstoff in einer Gewichtskonzentration von etwa 5 bis 500, insbesondere 10 bis 300 ppm enthalten. Die optimale Höhe der Konzentration des Wirkstoffs in dem Futter ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen oder speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineral-

Le A 22 397

stoffen enthalten. Das Futter kann sich beispielsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz und Spurenelementen.

Futterkonzentrate enthalten den Wirkstoff neben eßbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen in Konzentrationen von 0,5 - 90 Gew.-%, vorzugsweise 1 - 50 Gew.-%. Sie können nach den üblichen Mischmethoden hergestellt werden.

Vorzugsweise in Prämixen und Futterkonzentraten kann der Wirkstoff gegebenenfalls auch durch seine Oberfläche bedeckende geeignete Mittel, z.B. mit nichttoxischen Wachsen, Gelatine oder anderen Polymeren geschützt werden.

Beispiel für die Zusammensetzung eines Kükenaufzuchtfutters, das den erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

Le A 22 397

In einem kg Futtermischung sind enthalten:
600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4$ x $H_2O$, 140 mg $ZnSO_4$ x 7 $H_2O$, 100 mg $FeSO_4$ x 7 $H_2O$ und 20 mg $CuSO_4$ x 5 $H_2O$.

Beispiel für die Zusammensetzung eines Schweineaufzuchtfutters, das den erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischen 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

Le A 22 397

Patentansprüche:

1. 2-/N̄-(2-Hydroxyethyl)-carbamoyl̲7-3-methyl-chinoxa-
   lin-1,4-N-dioxid, dadurch gekennzeichnet, daß in der
   Korngrößenverteilung die Anzahl der Partikel mit
   einem Durchmesser von 0,02 bis 1 mm ein Maximum aufweist.

2. 2-/N̄-(2-Hydroxyethyl)-carbamoyl̲7-3-methyl-chinoxa-
   lin-1,4-N-dioxid nach Anspruch 1, dadurch gekennzeichnet, daß die Anzahl der Partikel mit einem
   Durchmesser von 0,02 bis 0,5 mm ein Maximum aufweist.

3. Verfahren zur Herstellung von 2-/N̄-(2-Hydroxyethyl)-
   carbamoyl̲7-3-methyl-chinoxalin-1,4-N-dioxid, dadurch
   gekennzeichnet, daß man

   a)   den Wirkstoff auf den Korngrößenbereich gemäß
        Anspruch 1 aus der Endlösung 2 ausfällt

   b)   den Wirkstoff auf den Korngrößenbereich gemäß
        Anspruch 1 mahlt

   c)   den Wirkstoff auf den Korngrößenbereich gemäß
        Anspruch 1 durch Agglomeration oder Granulation feinerer Kristalle einstellt.

4. Verwendung von 2-/N̄-(2-Hydroxyethyl)-carbamoyl̲7-
   3-methyl-chinoxalin-1,4-N-dioxid gemäß Anspruch 1
   zur Herstellung von Futtermittelzusätzen.

Le A 22 397

5.  Verwendung von 2-[N̄-(2-Hydroxyethyl)-carbamoyl]-
    3-methyl-chinoxalin-1,4-N-dioxid gemäß Anspruch 1
    zur Herstellung von Prämixen für die Futtermittelherstellung.

6.  Prämixe, Futtermittelzusätze und Futtermittel,
    enthaltend 2-[N̄-(2-Hydroxyethyl)-carbamoyl]-3-
    methyl-chinoxalin-1,4-N-dioxid gemäß Anspruch 1.

7.  Pulverförmige, stranggepreßte, pelletierte oder granulierte Futtermittel, enthaltend 2-[N̄-(2-Hydroxyethyl)-carbamoyl]-3-methyl-chinoxalin-1,4-N-dioxid
    gemäß Anspruch 1.

8.  Futtermittel in Form von Blöcken, enthaltend
    2-[N̄-(2-Hydroxyethyl)-carbamoyl]-3-methyl-chinoxalin-
    1,4-N-dioxid gemäß Anspruch 1.

9.  Prämixe, Futtermittelzusätze sowie Futtermittel
    gemäß den Ansprüchen 7 und 8, dadurch gekennzeichnet, daß sie 2-[N̄-(2-Hydroxyethyl)-carbamoyl]-3-
    methyl-chinoxalin-1,4-N-dioxid und weitere antibiotische und nicht-antibiotische ergotrope Substanzen
    enthalten.

10. Prämixe nach Anspruch 6 und 9, dadurch gekennzeichnet, daß sie 0,5 bis 90 Gew.-% 2-[N̄-(2-Hydroxyethyl)-
    carbamoyl]-3-methyl-chinoxalin-1,4-N-dioxid enthalten.

11. Futtermittel nach Anspruch 6 und 9, dadurch gekennzeichnet, daß sie 5 - 500 ppm, vorzugsweise 10 - 200 ppm 2-/N̄-(2-Hydroxyethyl)-carbamoy_l_7-3-methyl-chinoxalin-1,4-N-dioxid enthalten.

12. Futtermittel nach Anspruch 6, dadurch gekennzeichnet, daß es (2-/N̄-(2-Hydroxyethyl)-carbamoy_l_7-3-methyl-chinoxalin-1,4-N-dioxid und gegebenenfalls weitere antibiotische und nicht-antibiotische Ergotropica in ergotropen Dosen enthält.

Le A 22 397

FIG. 1

Le A 22 397

**0134444**

Nummer der Anmeldung

## Europäisches Patentamt
## EUROPÄISCHER RECHERCHENBERICHT

| EINSCHLÄGIGE DOKUMENTE | | EP 84107300.0 |
|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| Y | DE - A1 - 2 802 397 (HENKEL)<br><br>* Anspruch 1; Seite 2, Zeilen 13-30 *<br><br>-- | 1,4-12 | C 07 D 241/44<br>A 23 K    1/16 |
| Y | ULLMANNS ENCYKLOPÄDIE DER TECH-NISCHEN CHEMIE, 4. Auflage, Band 1, 1972, "Verfahrenstechnik I (Grundoperationen)"<br><br>VERLAG CHEMIE, Weinheim/Bergstr. Seiten 1,2<br><br>* Seite 1, Spalte 2, Zeilen 8-17 *<br><br>-- | 1,3 | |
| Y | K.WINNACKER, L. KÜCHLER, Chemische Technologie, 2. Auf-lage, Band 1, 1958, "Anorganische Technologie I"<br><br>CARL HANSER VERLAG, München Seiten 60,61<br><br>* Kapitel 2.22 *<br><br>---- | 1,3 | **RECHERCHIERTE SACHGEBIETE (Int. Cl.4)**<br><br>C 07 D 241/00<br>A 23 K |

Der vorliegende Recherchenbericht wurde für alle Patentansprüche erstellt.

| Recherchenort | Abschlußdatum der Recherche | Prüfer |
|---|---|---|
| WIEN | 16-10-1984 | LUX |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503 03 82